# EUROPEAN PATENT APPLICATION

(11) **EP 2 765 425 A1**
(43) Date of publication of application: **13.08.2014**
(21) Application number: 14153971.8
(22) Date of filing: 05.02.2014
(51) Int. Cl.: G01N 33/72

(54) **Support for capturing glycated protein in a sample and device and method for measuring the glycated protein using the support**

(30) Priority: 07.02.2013 KR 20130014126
(71) Applicant: Samsung Electronics Co., Ltd, Gyeonggi-do 443-742 (KR)
(72) Inventor: Lee, Woo-chang, Gyeonggi-do (KR); Kim, Sang-kyu, Gyeonggi-do (KR); Song, Kyung-mi, Gyeonggi-do (KR); Oh, Jin-mi, Gyeonggi-do (KR); Lee, Soo-suk, Gyeonggi-do (KR); Choi, Youn-suk, Gyeonggi-do (KR)
(74) Representative: Grünecker, Kinkeldey, Stockmair & Schwanhäusser

(57) **Abstract**

Provided is a support for effectively capturing glycated protein in a sample, and a device and method for measuring the glycated protein by using the support.

## Description

### BACKGROUND

### 1. Field

The present disclosure relates to a support for effectively capturing glycated protein in a sample, and a device and method for measuring the glycated protein using the same, as well as a method for preparing such support.

### 2. Description of the Related Art

Glycated hemoglobin refers to hemoglobin that is bound to a sugar. A saccharide can bind to a hemoglobin A chain. For example, glycated hemoglobin may include hemoglobin A1a (HbA1a), hemoglobin A1b (HbA1b), hemoglobin A1c (HbA1c) or a combination thereof. Among HbA1 a, HbA1 b and HbA1 c, HbA1 c in which glucose is bound to a valine residue at the N-terminal of β-chain has been known to account for about 60% to about 80% of the total glycated hemoglobin.

Glycated hemoglobin may serve as a good indicator to show a blood glucose level in a human body because glycated hemoglobin can reveal the average blood glucose concentration of a patient for the past 2-3 months. In general, the blood glucose level measured according to the conventional method may vary depending on whether it is measured on an empty stomach or after a meal. However, the method based on glycated hemoglobin may not be affected by the short-term variation regardless of the intake of a meal.

Accordingly, there is a desire for the development of a device or method for efficiently measuring the level of glycated protein in a sample.

### SUMMARY

According to an aspect of the present invention, there is provided a support for efficiently capturing glycated protein in a sample, wherein the support comprises a polymer containing a boronic acid moiety. According to one aspect of the invention, the support comprises a polymer foam containing boronic acid moieties. In another aspect of the invention, the support comprises a polymer foam and a carbohydrate polymer on a surface of the polymer foam, wherein the carbohydrate polymer comprises boronic acid moieties.

According to another aspect of the present invention, there is provided a chromatography column including the support for efficiently capturing glycated protein in a sample.

According to a further aspect of the present invention, there is provided a device for efficiently measuring the level of glycated protein in a sample. The device comprises (1) a first region including a support for capturing glycated protein, as provided herein, a first reaction chamber that is in fluid communication with the support, and a first detection area that is in fluid communication with the first reaction chamber; and (2) a second region including a second reaction chamber and a second detection area that is in fluid communication with the second reaction chamber.

According to a still further aspect of the present invention, there is provided a method for efficiently measuring the level of glycated protein in a sample. The method comprises injecting a sample including the glycated protein and non-glycated protein into the support for capturing the glycated protein provided herein; measuring a signal from non-glycated protein not bound to the support; and comparing the measured signal with a signal measured from a sample including both the glycated protein and the non-glycated protein.

According to a further aspect of the present invention, there is a method of preparing a support for capturing glycated protein, the method comprising providing a polymer support comprising interconnected, open-cell pores; and impregnating the support with a carbohydrate polymer, such that the carbohydrate polymer forms a thin film over the surface of the polymer support; wherein the carbohydrate polymer comprises boronic acid groups, or the method further comprises forming boronic acid groups in the carbohydrate polymer on the polymer support.

### BRIEF DESCRIPTION OF THE DRAWINGS

These and/or other aspects will become apparent and more readily appreciated from the following description of the embodiments, taken in conjunction with the accompanying drawings of which:
FIG. 1 is a diagram showing a method for measuring the level of glycated protein in a sample according to an embodiment of the present disclosure, wherein Hb=hemoglobin and gHb=glycated hemoglobin;
FIG. 2 is a drawing illustrating a precursor of a support including a boronic acid moiety according to an embodiment of the present disclosure;
FIG. 3 is a drawing illustrating a support including a boronic acid moiety according to an embodiment of the present disclosure;
FIG. 4 is a plane view of a device according to an embodiment of the present disclosure;
FIG. 5 is a cross-sectional view of a first region of a device according to an embodiment of the present disclosure;
FIG. 6 is a cross-sectional view of a second region of a device according to an embodiment of the present disclosure;
FIG. 7 is another cross-sectional view of a first region of a device according to an embodiment of the present disclosure;
FIG. 8 is a graph illustrating the absorbance spectra of calibrator sera passed through a support for capturing glycated hemoglobin according to an embodiment of the present disclosure, wherein absorbance (10 mm path) is indicated on the y-axis, and wavelength (nm) is indicated on the x-axis;
FIG. 9 is an absorbance ratio plot corresponding to the HbA1 c concentration. The absorbance ratio was calculated by the absorption values at specific wavelength of each control serum before and after passing through the support prepared by the embodiment of present disclosure. The Abs(HbA1c)/Abs(Total Hb) is indicated on the y-axis, and the HbA1 c concentration (%) is indicated on the x-axis; and
FIG. 10 is an absorbance ratio plot corresponding to the HbA1c concentration. The absorbance ratio was calculated by the absorbance value of each control serum measured by means of a device described in an embodiment of the present disclosure. The Abs(HbA1c)/Abs(Total Hb) is indicated on the y-axis, and the HbA1c concentration (%) is indicated on the x-axis.

### DETAILED DESCRIPTION

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list, i.e. "at least one of" encompasses and disclose each and every possible combination of one, two, etc. up to all elements in the list.

In an embodiment, the present disclosure provides a support for capturing a glycated protein comprising, consisting essentially of, or consisting of a polymer containing a boronic acid moiety, of a carbohydrate polymer containing a boronic acid moiety or a combination thereof.

The support may be a porous support, i.e., a porous support that has a plurality of pores inside the support. The support may be such that at least some part of pores may be interconnected enabling mutual fluid communication through the pores. The support may be such that at least some part of the pores in the support may be a type of open-cell pores that are mutually and continuously connected. The open-cell type support may refer to a support in which a liquid and/or a fluid can pass through the open-cell pores of the support. Absorption of liquid may be caused by the capillary force of the pores in the support.

As used herein, a polymer containing a boronic acid moiety may refer to a polymer which excludes or includes a carbohydrate polymer.

As used herein, a polymer containing a boronic acid moiety may refer to a polymer foam or foamable polymer containing the boronic acid moiety. The foam may be an open-cell foam. The support may be an open-cell type support or open-pore shaped article due to the pores of the polymer foam. The open-cell foam may include a foam that contains about 20% or more of open-cells as measured according to ASTM D2856-A. The open-pored shaped articles may be prepared according to foaming process with air or with other gases, and molding process (see example US 4,083,906; KR 10-2000-0065319).

Additionally or alternatively, the support may be a sponge-like material having a lattice defining a plurality of cavities. The support may absorb and/or retain a liquid through pores. By capillary phenomenon, the liquid may be absorbed and/or retained within the pores and/or cavities of the support. As used herein, the liquid may include a fluid.

The support may be a matrix. The support may be a separation matrix. The term used herein, "matrix" refers to a material including a porous solid support in which a polymer containing a boronic acid moiety, a carbohydrate polymer containing a boronic acid moiety or a combination thereof are attached thereto. The separation matrix in the chromatography field may often refer to a medium or resin. The separation matrix may be manufactured in any type that is conventionally used. The conventional types may include a monolith, a filter, a membrane, a chip, a capillary tube or a fiber.

The polymer containing the boronic acid moiety may be a sponge-like material. The sponge-like material may refer to a material having elasticity that enables the support to retain and/or discharge a liquid. The polymer containing the boronic acid moiety may be an elastic polymer. The elastic polymer may be an elastomer. The polymer may be also biocompatible. Additionally, the polymer may be a thermoplastic polymer. The thermoplastic polymer may be an injection moldable or moldable polymer. The thermoplastic polymer foam may be selected from the group consisting of polyvinyl alcohol (PVA), polyethylene (PE), polyurethane (PU), polyvinyl ether (PVE), polyolefin, polyester, polyamide, polyhydroxyethyl methacrylate (PHEMA), methylmethacrylate (MMA), N-vinyl pyrrolidone (N-VP) and a combination thereof.

The support may be liquid absorbing. The support may absorb a liquid through at least some of its pores. The support may absorb and/or retain a liquid within the open-cells of the support. In addition, the liquid retained by the support may be discharged. The liquid retained by the support may be discharged from the support by pressing the support. The pressing may be manually performed and/or automatically performed by an actuator. The actuator may be embodied so that it may deliver pressure to the support and/or impart pressure thereon. The liquid may be a sample including the glycated protein.

Furthermore, the polymer containing the boronic acid moiety may be a hydrophilic polymer. The polymer may include a polar group. The polymer group may include a hydroxyl group. The polymer may be polyhydroxy polymer. The polymer may be a polyhydroxy polymer including alternatively a polar (-CH-OH) group and a nonpolar (-CH₂-) group. The polymer may be a water-soluble polymer foam. The hydrophilic polymer foam may be selected from the group consisting of polyvinyl alcohol (PVA), polyurethane (PU), polyvinyl ether (PVE), polyester, ethyl vinyl acetate (EVA), polyamide, polyhydroxyethyl methacrylate (PHEMA), methylmethacrylate (MMA), N-vinyl pyrrolidone (N-VP) and a combination thereof.

The support may include a carbohydrate polymer containing a boronic acid moiety. The carbohydrate polymer may be one that is conventionally used for the support. The carbohydrate polymer may be selected from the group consisting of agarose, agar, cellulose, dextran, pectin, chitosan, konjac, carrageenan, gellan, alginate, alginic acid, starch and a combination thereof. The carbohydrate polymer may be a cross-linked carbohydrate polymer.

The polymer and/or carbohydrate polymer containing a boronic acid moiety may be a polymer and/or carbohydrate polymer in which a boronic acid moiety is functionalized. The functionalization of a boronic acid moiety may form a polymer with a functionalized boronic acid moiety and/or a carbohydrate polymer with a functionalized boronic acid moiety by oxidizing a hydroxyl group of the polymer and/or the carbohydrate polymer, and then reacting with a boronic acid derivative. The oxidization may change the hydroxyl group into a carbonyl or epoxy group. In an embodiment, the support may be formed by adding a hydrophilic polymer to the agarose.

The mean diameter of the open-cell pores of the support according to an embodiment of the present disclosure is in the range of about 1 µm to about 200 µm, for example, about 1 µm to about 150 µm, about 10 µm to about 150 µm, about 50 µm to about 150 µm, about 70 µm to about 150 µm, about 100 µm to about 150 µm, about 110 µm to about 150 µm, about 10 µm to about 110 µm, about 50 µm to about 110 µm, about 70 µm to about 110 µm, about 100 µm to about 110 µm, about 10 µm to about 100 µm, about 50 µm to about 100 µm, or about 70 µm to about 100 µm. The mean diameter of the open-cell pores of the support according to an embodiment of the present disclosure may be determined by a mean diameter of open-cell pores of a polymer foam and is smaller than the mean diameter of the pores of the polymer foam by about 5 to about 50%. The mean diameter of the open-cell pores may be measured by an imaging device such as an optical microscope.

The term "boronic acid moiety" used herein may refer to a moiety having dihydroxyboryl ((OH)₂B-). The compound including the boronic acid moiety may be (OH)₂B- or (OH)₂B-R₁-, where R₁ is a hydrocarbon having 1 to 30 carbon atoms. R₁ may, for example, be a linear or branched, saturated or unsaturated hydrocarbon having 1 to 30 carbon atoms. R₁ may, for example, include an aromatic hydrocarbon having 1 to 30 carbon atoms. R₁ may be methylene, ethylene, propylene, butylene, iso-butylene, pentylene, iso- pentylene or phenylene. The boronic acid moiety may or may not include a dye. The dye may, for example, be a fluorescent dye, a phosphorescent dye or a combination thereof. The dye may discharge a detectable signal that is distinguished from that of the non-glycated protein itself. For example, a dye may have an azo group.

In the support, the capturing may occur by a selective cis-diol binding between a boronic acid of the support including the boronic acid moiety and a saccharide of glycated protein. The selective binding may be a binding due to the affinity or a hydrogen bond between the boronic acid and the saccharide.

Referring to the support, "glycated protein" may refer to glycated polypeptide or glycated amino acid. "Glycated protein" may include, for example, glycated hemoglobin, a fragment of glycated hemoglobin, glycated amino acid, glycated polypeptide or a combination thereof. The glycated hemoglobin includes HbA1a, HbA1b, HbA1c or a combination thereof.

In another aspect, the present disclosure provides a method for manufacturing a support for capturing glycated protein including a polymer containing a boronic acid moiety, a carbohydrate polymer containing a boronic acid moiety or a combination thereof. The details on the support are the same as described above.

In an embodiment for manufacturing the support for capturing glycated protein, the polymer containing a boronic acid moiety may be a polymer foam. In an embodiment, the polymer foam may be manufactured by adding a blowing agent to a polymer that is not a polymer foam, see for example, Brian Bolto, Thuy Tran, Hanh Hoang, and Zongli Xie, Crosslinked poly(vinyl alcohol) membranes, Progress in polymer science 34(9), 969-981, 2009. The blowing agent used herein may be selected from the group consisting of aliphatic hydrocarbons such as butane, propane, isobutene, pentane, hexane and heptanes; cycloaliphatic hydrocarbons such as cyclobutane, cyclopentane and cyclohexane; and halogenated hydrocarbons such as chlorodifluorormethane, dichloromethane, dichlorofluoromethane, trichlorofluoromethane, chloroethane, dichlorotrifluoroethane and perfluorocyclobutane; and an inorganic gas such as carbon dioxide, nitrogen and air.

In a method for manufacturing a support for capturing glycated protein, the support may be manufactured by adding a carbohydrate polymer to a polymer. The polymer may be included within the carbohydrate polymer, thereby forming the support. The support may be formed by injecting the carbohydrate polymer into the open-cell type pores possessed by the polymer.

The support for capturing glycated protein including a polymer containing a boronic acid moiety, a carbohydrate polymer containing a boronic acid moiety or a combination thereof may be manufactured by functionalizing the hydroxyl group of the polymer and/or the carbohydrate polymer by using a boronic acid moiety. The functionalization of a boronic acid moiety can manufacture a polymer and/or a carbohydrate polymer with a functionalized boronic acid moiety by oxidizing the hydroxyl group of the polymer and/or the carbohydrate polymer and then reacting with a boronic acid derivative. The oxidization may be for oxidizing the hydroxyl group into a carbonyl or epoxy group.

In another aspect of the present disclosure, there is provided a chromatography column having a support including a polymer containing a boronic acid moiety, a carbohydrate polymer containing a boronic acid moiety or a combination thereof. The details on the support are described above. The column may be manufactured using any conventional material, for example, biocompatible plastic. The biocompatible plastic may be, for example, polypropylene, glass or stainless steel. The column may be in the size suitable for a lab scale or large scale purification.

In another aspect, there is provided a device for measuring glycated protein including: a first region including a support, a first reaction chamber that is in fluid communication with the support, and a first detection area that is in fluid communication with the first reaction chamber, wherein the support includes a polymer containing a boronic acid moiety, a carbohydrate polymer containing a boronic acid moiety or a combination thereof; and a second region including a second reaction chamber and a second detection that is in fluid communication with the second reaction chamber.

In the above device, the first detection area may be optically transparent. In the first detection area, a signal measuring device may be disposed to measure a signal from the glycated protein present in the area. For example, a device for measuring an optical signal, an electrical signal, a mechanical signal or a combination thereof may be disposed. The details on the support are the same as described above. The support may absorb a liquid and/or fluid. The diameter of the open-cell pores of the support is in the range of about 1 µm to about 200 µm, for example, about 1 µm to about 150 µm, about 10 µm to about 150 µm, about 50 µm to about 150 µm, about 70 µm to about 150 µm, about 100 µm to about 150 µm, about 110 µm to about 150 µm, about 10 µm to about 110 µm, about 50 µm to about 110 µm, about 70 µm to about 110 µm, about 100 µm to about 110 µm, about 10 µm to about 100 µm, about 50 µm to about 100 µm, or about 70 µm to about 100 µm.

A support for capturing glycated protein according to the present invention may be installed in the first region. The support may include a polymer containing a boronic acid moiety, a carbohydrate polymer containing a boronic acid moiety or a combination thereof. The support may be connected to the first reaction chamber that is in fluid communication with the support. The support may be disposed in a first inlet. The support may be connected to the first inlet. The second region may not include the support.

In an embodiment, the device may be a microfluidic device in which an inlet and an outlet may be connected via a channel or chamber. The microfluidic device may include at least one channel or chamber with a cross-sectional length of about 1 µm to about 1000 µm for example, about 10 µm, about 50 µm, about 100 µm, about 200 µm, about 300 µm, about 400 µm, about 500 µm, about 600 µm, about 700 µm, about 800 µm, or about 900 µm. When the cross-section is spherical, its length refers to its diameter.

In the device, the second region may not include the support. The device may further include an actuator, which imparts pressing on the support. The actuator may deliver and/or add pressure on the support. The actuator may be configured to add pressure on the support so that the liquid retained in the support may be discharged from the support. The support may be, for example, a pusher or a presser.

In an embodiment, the reaction chamber or the detection area may include a reagent for selectively confirming the presence of glycated protein. The reagent may be a glycated protein discoloring reagent. The reagent may be applied on the reaction chamber or the detection area. The reagent may be a solidified reagent or a dried reagent. The discoloring reagent for glycated protein may be glycated hemoglobin or hemoglobin discoloring reagent. The glycated hemoglobin or hemoglobin discoloring reagent may be suitably selected by one of ordinary skill in the art. Examples of the discoloring reagent include potassium ferricyanide (K₃Fe(CN)₆) and potassium cyanide (KCN), or potassium ferricyanide (K₃Fe(CN)₆) and lithium thionecyanide (LiSCN). The device may be a disposable and/or a portable device.

In another aspect, the present disclosure provides a method for measuring glycated protein in a sample including: forming a support bound to glycated protein by injecting a sample including glycated protein into the support for capturing glycated protein according to the present invention, in which the support includes a polymer containing a boronic acid moiety, a carbohydrate polymer containing a boronic acid moiety or a combination thereof; measuring a signal from non-glycated protein not bound to the support; and comparing the measured signal with the signal measured from a sample including both glycated protein and non-glycated protein.

The method includes injecting a sample including glycated protein into a support for capturing glycated protein, in which the support includes a polymer containing a boronic acid moiety, a carbohydrate polymer containing a boronic acid moiety or a combination thereof, thereby forming a support wherein the glycated protein is bound thereto. The formation of the support wherein the glycated protein is bound thereto may be performed by incubating a support including a boronic acid moiety and a sample including glycated protein. The injection or incubation may be performed under a condition that can induce a cis-diol binding due to affinity between boronic acid and a saccharide, for example, incubating at room temperature without stirring.

The method includes measuring a signal from a non-glycated protein not bound to the support. The measurement of the signal may include a measurement of an optical signal, an electrical signal, a mechanical signal, or a combination thereof. The measurement of the signal may be the measurement of the non-glycated protein itself. For example, the glycated protein may be a glycated hemoglobin, and the measurement of the signal may be the measurement of the non-glycated hemoglobin-specific optical signal. For example, the measurement may be the measurement of glycated or non-glycated hemoglobin-specific absorbance, for example, the absorbance in the range of about 400 nm to about 430 nm (e.g., about 405 nm, about 410 nm, about 415 nm, about 420 nm, or about 425 nm).

The method includes comparing the measured signal with a signal measured from a sample including both a glycated protein and a non-glycated protein. The measurement of the signal may include a measurement of an optical signal, an electrical signal, a mechanical signal or a combination thereof. The measurement of the signal may be the measurement of a glycated protein itself, a non-glycated protein itself or a combination thereof. For example, the glycated protein may be a glycated hemoglobin, and the measurement of the signal may be the measurement of a glycated hemoglobin-specific optical signal and a non-glycated hemoglobin-specific optical signal. For example, the measurement may be the measurement of hemoglobin-specific absorbance, for example, the absorbance in the range of about 400 nm to about 430 nm (e.g., about 405 nm, about 410 nm, about 415 nm, about 420 nm, or about 425 nm).

The measurement of a signal from a non-glycated protein and the measurement of a signal from a sample including both a glycated protein and a non-glycated protein may be performed concurrently or sequentially. Furthermore, the measurement of a signal from a non-glycated protein and the measurement of a signal from a sample including both a glycated protein and a non-glycated protein may be performed in the same device. The device may be one for measuring the glycated protein.

The method may further include pressing on the support. The pressing may be performed manually or through an actuator included in the device. The details of the actuator are described above. The pressing may impart a pressure on part of the support, thereby discharging a hemolytic solution retained in the support. The hemolytic solution retained in the support, excluding the glycated hemoglobin bound to the boronic acid moiety of the support, may be discharged through the pressing.

Conventional assay methods quantitatively measure HbA1c by multi-step reactions, typically requiring a mixing process prior to quantification. In an additional aspect of the invention, the support, chromatography column, device, and method of the present disclosure can be employed without providing an apparatus for a washing process or including an additional external mixing process.

According to another aspect of the present invention, the support of the present disclosure may be used to efficiently capture a glycated protein in a sample. Furthermore, the disclosed support may efficiently retain a sample including the glycated protein and discharge a liquid excluding the glycated protein retained in the support by applying pressure on the support.

According to an aspect of the present invention, the chromatography column including a support of the present disclosure may efficiently separate glycated protein in a sample from non-glycated protein and/or other components of the sample.

According to an aspect of the present invention, the device of the present disclosure may be used to efficiently measure the level of glycated protein in a sample (e.g., determine a ratio of glycated to non-glycated protein in a sample). In addition, the device may separately measure the signal of a non-glycated protein in the sample from the signal of the total protein in the sample, and, optionally, compare the two signals to determine the relative amount of glycated protein in the sample.

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein.

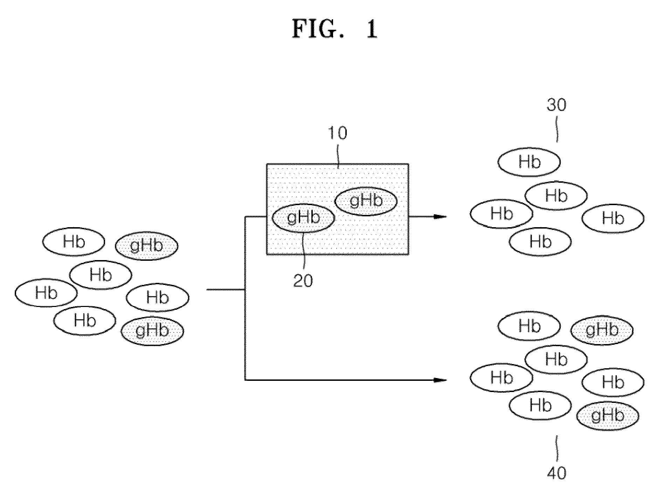

FIG. 1 diagrammatically shows a method for measuring the level of glycated protein in a sample according to an embodiment of the present disclosure. As shown in FIG. 1, glycated hemoglobin (gHb) 20 may be selectively removed from a combination of glycated hemoglobin (gHb) and non-glycated hemoglobin (Hb) by contacting the combination with the support 10 including a boronic acid moiety. As a result, a signal OD2 measured from a mixture 40 of glycated hemoglobin (gHb) and non-glycated hemoglobin (Hb), and a signal OD1 measured from non-glycated hemoglobin (Hb) 30 may be obtained, and the concentration of the glycated hemoglobin in a sample from OD2 and OD1 may also be obtained.

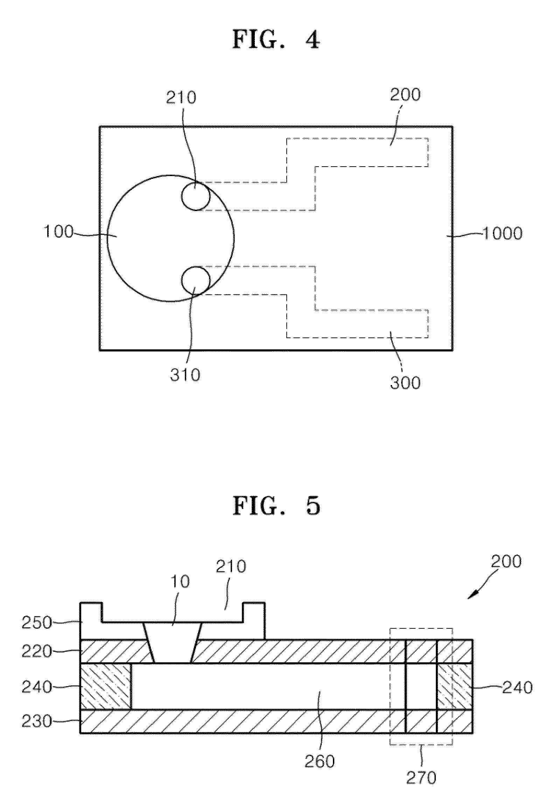

FIG. 4 shows a plane view of a device according to an embodiment of the present disclosure. As shown in FIG. 4, a device 1000 for measuring the level of a glycated protein may include a first region 200 and a second region 300, and may optionally include a sample inlet 100. The first region 200 and/or the second region 300 may include a first inlet 210 and/or a second inlet 310 through which a sample is respectively introduced into the first region 200 and/or the second region 300. A sample including the glycated protein introduced through a sample inlet 100 may be introduced into the first region 200 and the second region 300 through the first inlet 210 and the second inlet 310, respectively. The device for measuring the level of glycated protein may be a cartridge.

FIG. 5 shows a cross-sectional view of a first region of a device according to an embodiment of the present disclosure. As shown in FIG. 5, the first region 200 may include the support 10, a first reaction chamber 260 that is in fluid communication with the support 10, and a first detection area 270 that is in fluid communication with the first reaction chamber 260. The first reaction chamber 260 may be an internal cavity surrounded by a first substrate 220, a second substrate 230 which faces the first substrate 220, and a spacer 240 disposed in between the first substrate 220 and the second substrate 230. A third substrate 250 may be disposed on a top surface of the first substrate 220. The third substrate 250 may further include a projection on the upper part of the third substrate 250. The first substrate 220 and the third substrate 250 may each include a hole, wherein the hole in the first substrate 220 and the hole in the third substrate 250 overlap. The shape of the hole may be in any form. The support 10 may be disposed in the hole. The support 10 may be formed to fit into the hole. The support 10 may be disposed in the hole so that it may completely fill in the hole formed in a part of the first substrate 220 and the third substrate 250, respectively. A first inlet 210 may be formed through the third substrate 250, which includes the support 10 and the projection. A liquid introduced through the first inlet 210 may be absorbed by the support 10. The liquid may be a sample including a glycated protein. The sample including the glycated protein may be absorbed by the support 10 through the first inlet 210. As described above, in the support 10, the glycated protein may be attached to the support 10 through a boronic acid moiety included in the support 10.

The material of a substrate that respectively surrounds the top and bottom parts of the first detection area 270 may be light transmitting. The material of the substrate that respectively surrounds the top and bottom parts of the first detection area 270 may be different from those of the first substrate and the second substrate.

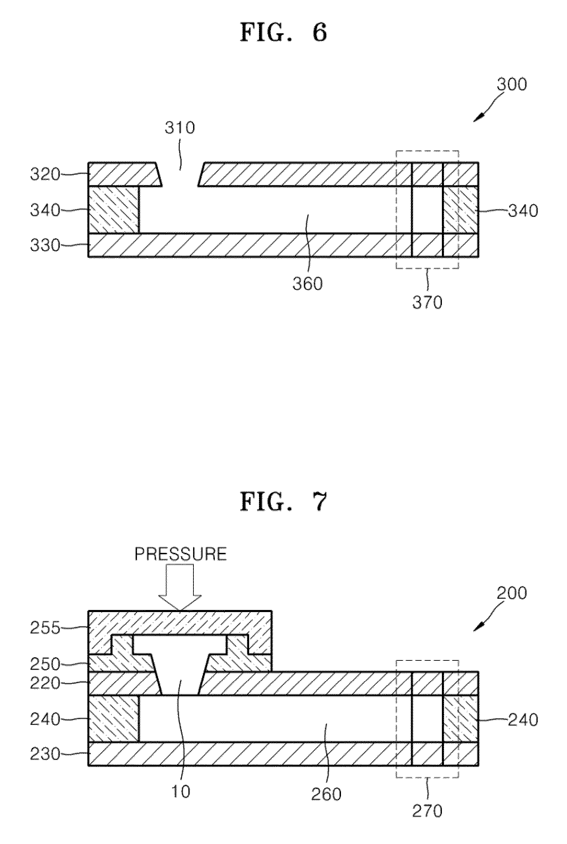

FIG. 6 shows a cross-sectional view of a second region of a device according to an embodiment of the present disclosure. As shown in FIG. 6, the second region 300 may include a second detection area 370 that is fluid communication with the second reaction chamber 360. The second reaction chamber 360 may be an internal cavity surrounded by a first substrate 320, a second substrate 330 which faces the first substrate 320, and a spacer 340 disposed in between the first substrate 320 and the second substrate 330. Optionally, a third substrate (not shown) may be disposed on a top surface of the first substrate 320. The third substrate may further include a projection on the upper part of the third substrate. The first substrate 320 and selectively the third substrate may each include a hole, wherein the hole in the first substrate 320 and the hole in the third substrate overlap. The shape of the hole may be in any form. A second inlet 310 may be formed through the hole. The liquid introduced through the second inlet 310 can reach the second detection area 370 through the second reaction chamber 360. The sample may include a glycated protein. A second region may not include a means to separate the glycated protein. The means may be a support according to an embodiment of the present disclosure. A signal may be measured in the second detection area 370 from a sample including both a glycated protein and a non-glycated protein. The measurement of the signal may be the measurement of glycated protein-specific or non-glycated protein-specific absorbance, for example, the absorbance in the range of about 400 nm to about 430 nm (e.g., about 405 nm, about 410 nm, about 415 nm, about 420 nm, or about 425 nm).

FIG. 7 shows a cross-sectional view of a first region 200 of a device according to an embodiment of the present disclosure. As shown in FIG. 7, the first region 200 may include the support 10, a first reaction chamber 260 which is fluid communication with the support, and a first detection area 270 which is fluid communication with the first reaction chamber, and a lead 255. A third substrate 250 may further include a projection on the upper part of the third substrate. As a pressure is applied to the support, in the shown embodiment to the lead 255, the portion of a sample that is not captured by the support 10 may be discharged from the support 10 to which the sample previously was applied. The sample discharged from the support 10 may include a non-glycated protein only, while the glycated protein is bound to the support. The sample discharged from the support 10 may pass through the first reaction chamber 260 and reach the first detection area 270. The concentration of the non-glycated protein may be measured by measuring the signal of the non-glycated protein that arrived at the first detection area 270. The measured signal may be an optical signal, an electrical signal, a mechanical signal or a combination thereof. In an embodiment, the non-glycated protein may be a non-glycated hemoglobin, and the measurement of the signal may be the measurement of a non-glycated hemoglobin-specific optical signal. The measurement of the signal may be a non-glycated hemoglobin-specific absorbance, for example, the absorbance in the range of about 400 nm to about 430 nm (e.g., about 405 nm, about 410 nm, about 415 nm, about 420 nm, or about 425 nm).

### EXAMPLES

### Example 1: Method of manufacturing a polymer support containing a boronic acid moiety

A polymer support comprising hydrophilic polyvinyl alcohol (PVA), which can retain a mean pore size of about 100 µm or less by a foaming process, was prepared from Brushtech, Inc., Korea. The PVA support was an open-cell type enabling smooth movement of a liquid. The PVA support served as a sub-structure or scaffold for the final support.

The polymer support was coated with agarose. More specifically, the PVA support was added into an about 1% agarose solution while heating it at about 70 °C or above. Then, Bubble present inside the support were removed and the agarose solution was allowed to impregnate the support. The impregnated support was dried at about 45 °C for more than about 8 hours so that the surface of the PVA support, including the interior surfaces of the pores, was allowed to become coated with agarose. The thus prepared agarose coated PVA support showed improved hydrophilicity in comparison to a PVA support not coated with agarose, and the support was washed at least three times with deionized water to remove the residue.

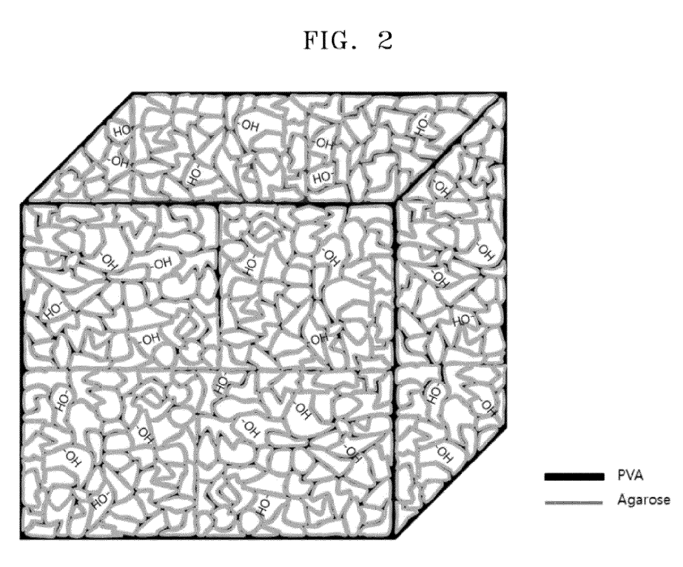

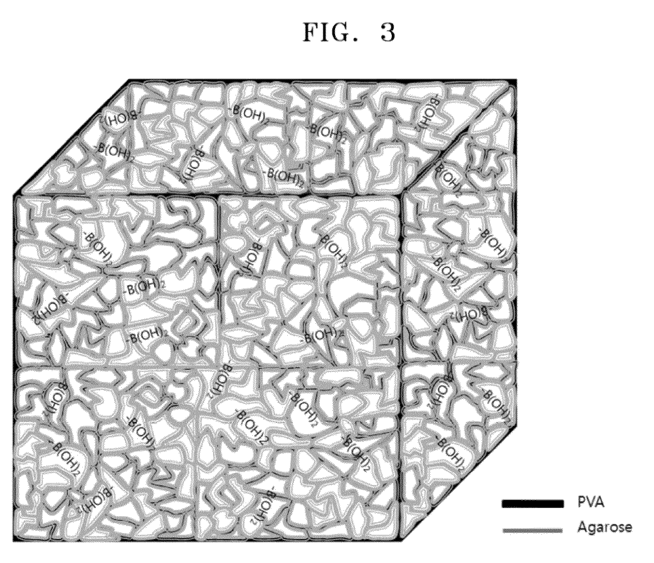

FIG. 2 shows a drawing illustrating a precursor of a support including a boronic acid moiety according to an embodiment of the present disclosure. As shown in FIG. 2, polymers or polymer foams are represented by lines inside a carbohydrate polymer formed in a quadrangle. The polymer or polymer foam or a carbohydrate polymer includes a hydroxyl group. As shown in FIG. 3, the hydroxyl group may be functionalized into a boronic acid moiety.

In order to functionalize the agarose surface formed on the PVA support in a thin film to a boronic acid, the hydroxyl groups present on the agarose surface were substituted with aldehyde groups using sodium periodate. The aldehyde groups present on the agarose surface were introduced with aminophenyl boronic acid so that the boronic acid can be bound to the agarose surface.

FIG. 3 shows a drawing illustrating a support including a boronic acid moiety according to an embodiment of the present disclosure. As shown in FIG. 3, the illustrated support for capturing a glycated protein includes a polymer containing a boronic acid moiety, a polymer foam, a carbohydrate polymer or a combination thereof. The support may be formed by functionalizing the hydroxyl group in the support as shown in FIG. 2 into a boronic acid moiety. In addition, methods for functionalization of a boronic acid moiety that are known in the art may be appropriately employed.

A sample including the glycated protein may be retained through the pores forming the open cells of the support. The support may retain a liquid via the interaction between the liquid molecules within the pores, and the mutual interaction between the surface of the liquid and the surface of the support including the pores. The pores forming the open cells of the support may retain a liquid via capillary phenomenon. The liquid may be a sample including the glycated protein. The sample including the glycated protein may be a hemolytic solution.

Since the support employs capillary phenomenon, it may not require an additional mixing between a sample including a boronic acid moiety and a sample including the glycated protein. Furthermore, through numerous pores forming the open cells of the support, the number of boronic acid moieties per unit volume of the support that can capture glycated protein can be increased.

The presence of capturing HbA1c using the polymer support prepared in Example 1 was measured. The HbA1 c calibrate (calibrator) #3 (HbA1 c 11.9%, IVD Lab., Korea) was diluted 1:100, and the polymer support was impregnated with an equal volume of 120 µL, respectively. After about 3 minutes, the polymer support was added into a syringe and pressed to release the hemolytic solution introduced therein.

Absorbance of the sample (calibrator #3) passed through the polymer support was measured with UV/VIS. spectrophotometer (AvaSpec 2048, Avantes BV, Apeldoorn, The Netherlands). Compared with the absorbance of an intact support, the absorbance was decreased owing to the capture of glycated hemoglobin in the support. The color of the prepared support turned red after separating the retained sample. When the same sample was applied to the polymer support without boronic acid moiety (just coated with agarose), its degree of color change was not so significant as with the support functionalized with boronic acid.

### Example 2: Measurement of the level of glycated hemoglobin using a polymer support containing a boronic acid moiety

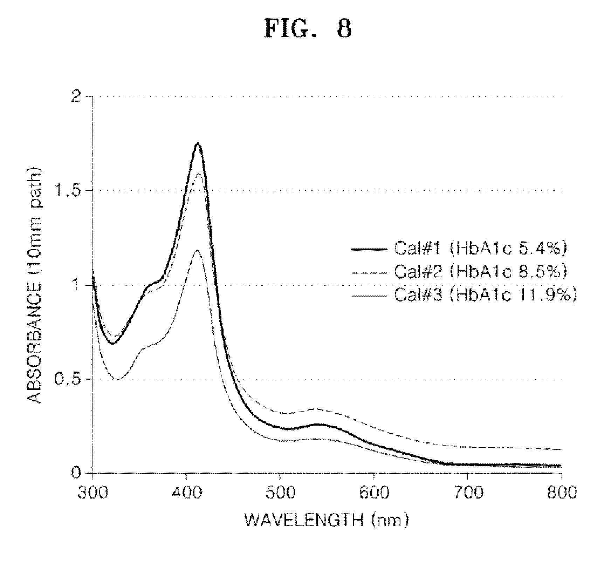

Glycated hemoglobin was measured using the polymer support prepared in Example 1 as follows. Specifically, HbA1 c calibrator #1 (HbA1 c 5.4%, IVD Lab., Korea), HbA1 c calibrator, #2 (HbA1 c 8.5%, IVD Lab., Korea), and HbA1 c calibrator #3 (HbA1 c 11.9%, IVD Lab., Korea) were each diluted about 1:100, and the polymer support was impregnated with an equal volume of about 120 µL, respectively. After about 3 minutes, the polymer support was added into a syringe, and pressed to release the hemolytic solution introduced therein. The result of the absorbance measured from the released hemolytic solution is shown in FIG. 8.

FIG. 8 shows a graph illustrating the absorbance of HbA1c according to the concentration of HbA1 c measured using a support for capturing glycated hemoglobin according to an embodiment of the present disclosure. In FIG. 8, absorbance of HbA1c according to HbA1c about 5.4%, about 8.5% and about 11.9% is presented.

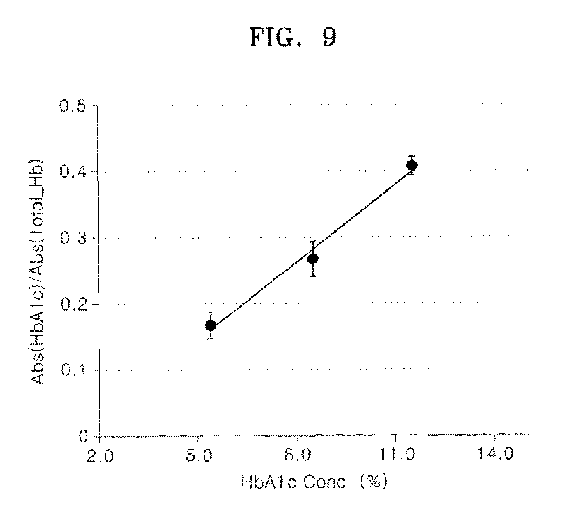

FIG. 9 displays an absorbance ratio plot corresponding to the HbA1c concentration in the range between about 5.4 and about 11.9%. Absorbance ratio was defined as the absorbance of the captured glycated proteins retained in the support to the absorbance of total hemoglobin. The absorbance of the numerator (captured glycated proteins) was calculated by means of subtracting the absorbance of the sample passed through the support from that of total hemoglobin. The support was prepared in accordance with an embodiment of the present disclosure. As shown in FIG. 9, the calculated absorbance ratio could be linearly related with the portion of HbA1 c in the hemolysate.

### Example 3: Measurement of the level of glycated hemoglobin using a device including a polymer support containing a boronic acid moiety according to an embodiment of the present disclosure

The level of HbA1c was measured using a device for measuring the level of glycated hemoglobin according to an embodiment of the present disclosure. A hemolytic solution was prepared by mixing whole blood collected from a tube in a constant ratio of about 25:1 to about 100:1 with a hemolytic buffer solution containing about 0.25 M ammonium acetate buffer containing about 40 mM MgCl₂ (pH about 8.9), about 0.06% SDS and about 0.07% Triton X-100. The hemolytic solution was introduced into the inlet of a device of the present disclosure for measuring the level of glycated hemoglobin. The hemolytic solution was absorbed into the polymer support substituted with a boronic acid and introduced into the inlet. After about 3-5 minutes, the HbA1c present in the hemolytic solution could be captured by the boronic acid present on the surface of the polymer support. As an external pressure is applied onto the support containing the hemolytic solution by an actuator, the liquid was forced to elute from the support matrix and to flow along a film-type chamber installed inside the device. Then, the absorbance of the liquid reaching the detection area may be measured using a measurement device and the concentration of the non-glycated hemoglobin can be calculated therefrom. In addition, in the second detection area, a signal was measured using the same method and the concentration of total hemoglobin was calculated therefrom.

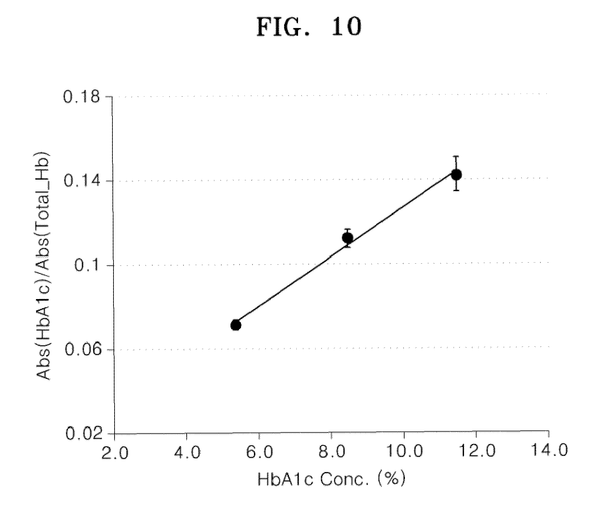

FIG. 10 displays an absorbance ratio corresponding to the concentration of HbA1 c. The absorbance ratio defined was proportional to the concentration of HbA1c measured with a commercial instrument (Tosoh G8, Japan). A linear relationship was also observed in the concentration range of about 5.5 to about 11.5% HbA1c.

It should be understood that the exemplary embodiments described herein should be considered in a descriptive sense only and not for purposes of limitation. Descriptions of features or aspects within each embodiment should typically be considered as available for other similar features or aspects in other embodiments.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein. Moreover, any combination of the above-described elements in all possible variations thereof is encompassed by the invention unless otherwise indicated herein or otherwise clearly contradicted by context.

## Claims

1. A support for capturing a glycated protein, the support comprising a polymer containing a boronic acid moiety.

2. The support according to claim 1, wherein the polymer containing the boronic acid moiety is a polymer foam containing a boronic acid moiety, a carbohydrate polymer containing a boronic acid moiety, or combination thereof, wherein the carbohydrate polymer is preferably selected from the group consisting of agarose, agar, cellulose, dextran, pectin, chitosan, konjac, carrageenan, gellan, alginate, alginic acid, starch and a combination thereof.

3. The support according to claim 1, wherein the support comprises a foam polymer coated with a carbohydrate polymer that comprises boronic acid moieties.

4. The support according to any one of claims 1 to 3, wherein the support has a plurality of pores inside the support and at least some part of pores are interconnected and allows fluid communication through the pores.

5. The support of claim 3, wherein the foam polymer comprises pores, and the carbohydrate polymer comprising boronic acid moieties is coated on the foam polymer within the pores.

6. The support according to claims 4 or 5, wherein the pores are open-cell pores that are interconnected, the average diameter of the open-cell pores preferably being in the range of 1 µm to 200 µm.

7. The support according to any one of claims 1 to 6, wherein the polymer containing the boronic acid moiety is hydrophilic.

8. The support according to any one of claims 1 to 7, wherein the polymer containing the boronic acid moiety is selected from the group consisting of polyvinyl alcohol (PVA), polyethylene (PE), polyurethane (PU), polyvinyl ether (PVE), polyolefin, polyester, ethyl vinyl acetate (EVA), polyamide, polyhydroxyethyl methacrylate (PHEMA), methylmethacrylate (MMA), N-vinyl pyrrolidone (N-VP) and a combination thereof.

9. The support according to any one of claims 1 to 8, wherein the polymer containing the boronic acid moiety is a sponge-like material having a lattice defining a plurality of cavities.

10. A device for measuring glycated protein, comprising:
a first region including a support according to any one of claims 1 to 9, a first reaction chamber that is in fluid communication with the support, and a first detection area that is in fluid communication with the first reaction chamber; and
a second region including a second reaction chamber, and a second detection area that is in fluid communication with the second reaction chamber.

11. The device according to claim 10, wherein the second region does not include the support.

12. The device according to claim 10 or 11, further comprising an actuator which applies pressure to the support.

13. The device according to any one of claims 10 to 12, wherein the first or second reaction chamber or the first or second detection area includes a reagent for quantization of glycated protein.

14. A method of preparing a support for capturing glycated protein, the method comprising:
providing a polymer support comprising interconnected, open-cell pores; and
impregnating the support with a carbohydrate polymer, such that the carbohydrate polymer forms a thin film over the surface of the polymer support;
wherein the carbohydrate polymer comprises boronic acid groups, and/or the method further comprises forming boronic acid groups on the carbohydrate polymer after impregnating the support with the carbohydrate polymer.

15. The method of claim 14, wherein:
- the polymer support comprising interconnected, open-cell pores is a polymer foam, and/or
- the carbohydrate polymer is agarose.
